# EUROPEAN PATENT APPLICATION

(11) **EP 1 837 346 A2**
(43) Date of publication of application: **26.09.2007**
(21) Application number: 07251157.9
(22) Date of filing: 20.03.2007
(51) Int. Cl.: C07K 14/535

(54) **Method for purifying granulocyte-colony stimulating factor**

(30) Priority: 21.03.2006 GB 0605684
(71) Applicant: Sicor Biotech UAB, 02241 Vilnius (LT)
(72) Inventor: Bumelis, Vladas Algirads, 10103 Vilnius (LT); Loreta, Janeniene, 14269 Vilnius (LT); Pesliakas, Jonas Henrikas, 04121 Vilnius (LT); Rimkeviciene, Jurate (J rat Rimkevi ien), Vilnius 03123 (LT); Vaitkevicius, Arunas, Vilnius 07103 (LT); Zunda, Giedrius, Vilnius 05123 (LT); Zvirblis, Gintautas, Vilnius 06111 (LT)
(74) Representative: Nachshen, Neil Jacob

(57) **Abstract**

The present invention provides a novel process for isolating and purifying rmetHuG-CSF from a G-CSF producing microorganism. The invention also relates to a method of improved over-expression of G-CSF in *E. coli.,* a primer sequence for amplification of a modified hG-CSF sequence, plasmids, expression vectors and host cells for use in such an improved method.

SEQUENCE LISTING

<110>  Sicor Biotech UAB

<120>  Method for purifying granulocyte-colony stimulating factor.

<130>  P024624WO

<150>  GB 0605684.0
<151>  2006-03-21

<160>  11

<170>  PatentIn version 3.4

<210>  1
<211>  27
<212>  DNA
<213>  Artificial

<220>
<223>  Isolated nucleic acid molecule

<400>  1
ctgcatatga cacctttagg acctgct
27


<210>  2
<211>  18
<212>  DNA
<213>  Artificial

<220>
<223>  Oligonucleotide primer

<400>  2
acccccctgg gccctggc
18


<210>  3
<211>  18
<212>  DNA
<213>  Artificial

<220>
<223>  Oligonucleotide primer

<400>  3
tcagggctgc gcaaggtg
18


<210>  4
<211>  18
<212>  DNA
<213>  Homo sapiens

<400> 4
   acccccctgg gccctgcc
   18

<210> 5
<211> 6
<212> PRT
<213> Homo sapiens

<400> 5

Thr Pro Leu Gly Pro Ala
1               5

<210> 6
<211> 21
<212> DNA
<213> Artificial

<220>
<223> Modified G-CSF sequence

<400> 6
atgacacctt taggacctgc t
21

<210> 7
<211> 7
<212> PRT
<213> Homo sapiens

<400> 7

Met Thr Pro Leu Gly Pro Ala
1               5

<210> 8
<211> 27
<212> DNA
<213> Artificial

<220>
<223> Oligonucleotide primer

<400> 8
ctgcatatga cacctttagg acctgct
27

<210> 9
<211> 21
<212> DNA
<213> Artificial

<220>

<223>  Oligonucleotide primer

<400>  9
ctgggatcct tatcagggct g
21


<210>  10
<211>  565
<212>  DNA
<213>  Artificial

<220>
<223>  Modified HuG-CSF sequence

<400>  10
catatgacac ctttaggacc tgctagctcc ctgccccaga gcttcctgct caagtgctta
60

gagcaagtga ggaagatcca gggcgatggc gcagcgctcc aggagaagct gtgtgccacc
120

tacaagctgt gccaccccga ggagctggtg ctgctcggac actctctggg catcccctgg
180

gctcccctga gctcctgccc cagccaggcc ctgcagctgg caggctgctt gagccaactc
240

catagcggcc ttttcctcta ccaggggctc ctgcaggccc tggaagggat atcccccgag
300

ttgggtccca ccttggacac actgcagctg gacgtcgccg actttgccac caccatctgg
360

cagcagatgg aagaactggg aatggcccct gccctgcagc ccacccaggg tgccatgccg
420

gccttcgcct ctgctttcca gcgccgggca ggaggggtcc tggttgctag ccatctgcag
480

agcttcctgg aggtgtcgta ccgcgttcta cgccaccttg cgcagccctg ataaggatcc
540

gaattcgagc tccgtcgaca agctt
565


<210>  11
<211>  175
<212>  PRT
<213>  Artificial

<220>
<223>  Modified HuG-CSF sequence

<400>  11

Met Thr Pro Leu Gly Pro Ala Ser Ser Leu Pro Gln Ser Phe Leu Leu
1               5                   10                  15

Lys Cys Leu Glu Gln Val Arg Lys Ile Gln Gly Asp Gly Ala Ala Leu
            20                  25                  30

Gln Glu Lys Leu Cys Ala Thr Tyr Lys Leu Cys His Pro Glu Glu Leu
        35                  40                  45

Val Leu Leu Gly His Ser Leu Gly Ile Pro Trp Ala Pro Leu Ser Ser
    50                  55                  60

Cys Pro Ser Gln Ala Leu Gln Leu Ala Gly Cys Leu Ser Gln Leu His
65                  70                  75                  80

Ser Gly Leu Phe Leu Tyr Gln Gly Leu Leu Gln Ala Leu Glu Gly Ile
                85                  90                  95

Ser Pro Glu Leu Gly Pro Thr Leu Asp Thr Leu Gln Leu Asp Val Ala
            100                 105                 110

Asp Phe Ala Thr Thr Ile Trp Gln Gln Met Glu Glu Leu Gly Met Ala
        115                 120                 125

Pro Ala Leu Gln Pro Thr Gln Gly Ala Met Pro Ala Phe Ala Ser Ala
    130                 135                 140

Phe Gln Arg Arg Ala Gly Gly Val Leu Val Ala Ser His Leu Gln Ser
145                 150                 155                 160

Phe Leu Glu Val Ser Tyr Arg Val Leu Arg His Leu Ala Gln Pro
                165                 170                 175

## Description

The present invention provides a method for purifying granulocyte-colony stimulating factor ("G-CSF") produced from recombinant sources. More particularly, the present invention relates to procedures for rapid and efficient isolation and purification of biologically active G-CSF produced from a transformed E. coli microorganism. The present invention also relates to a synthetic gene coding for human G-CSF for the expression in E. coli.

### BACKGROUND TO THE INVENTION

Granulocyte colony-stimulating factor (G-CSF) is a hematopoietic cytokine, released mainly by mononuclear cells and fibroblasts, that stimulates the proliferation, differentiation, and activation of cells of the granulocyte lineage into functionally mature neutrophils (L.M. Hollingshead, K.L. Goa. Recombinant granulocyte colony-stimulating factor (rG-CSF): a review of its pharmacological properties and prospective role in neutropenic conditions. Drugs, 42 (1991) 300-330).

The human form of G-CSF is a glycoprotein mediator composed of a single polypeptide chain of 174 amino acids coded for by a gene on chromosome 17 (J.E. Layton. Granulocyte colony-stimulating factor: structure, function and physiology. Growth factors, 6 (1992) 179-186)

After its purification (K. Welte, E. Platzer, L. Lu, J.L. Gabrilove, E.Levi, R. Mertelsmann, M.A.S. More. Purification and biochemical characterization of human pluripotent hematopoietic colony-stimulating factor. Proc. Natl. Acad. Sci. USA, 82 (1985) 1526-1530), the cloning and expression of cDNA for human granulocyte colony-stimulating factor was done by Nagata et al. (S. Nagata, M. Tsuchiya, T. Asano, Y. Kaziro, T. Yamazaki, O. Yamamoto, Y. Hirata, N. Kubota, M. Oheda, H. Nomura and M. Ono. Molecular cloning and expression of cDNA for human granulocyte colony-stimulating factor. Nature, 319, 415-418 (1986)), and by Souza et al. ( L.M. Souza, T.C. Boone, J. Gabrilove, P.H. Lai, K.M. Zsebo, D.C. Murdock, V.R. Chazin, J. Bruszewski, H. Lu, K.K. Chen, J. Barendt, E. Platzer, M.A.S. Moore, R. Mertelsmann and K. Welte. Recombinant human granulocyte colony-stimulating factor: effects on normal and leukemic myeloid cells. Science, 232 (1986) 61-65).

S. Nagata et al, have found G-CSF cDNA which codes for a protein consisting of 207 amino acids of which 177 code for mature G-CSF and also reported for cDNA for G-CSF that coded shorter form of G-CSF having a three amino acid deletion. Souza et al. reported on the cloning of the gene for hG-CSF and expression in Escherichia coli. In both cases the conformity of biological function of expressed G-CSF to human G-CSF was unambiguously established.

EP0215126 and EP0220520 describe genes coding for G-CSF and recombinant vectors for expression and production of a glycoprotein having G-CSF activity. US4810643 describes DNA sequences coding for all or part of hpG-CSF.

US499291 also describes G-CSF which may be the product of prokaryotic or eukaryotic host expression of an exogenous DNA sequence. Similarly, US6379661 describes the incorporation of sequences coding for part or all of the sequence of amino acid residues of hpG-CSF or for analogs thereof into autonomously replicating plasmid or viral vectors employed to transform or transfect suitable prokaryotic or eukaryotic host cells such as bacteria, yeast or vertebrate cells in culture.

The availability of human G-CSF cDNA initiated the production of large quantities of hG-CSF in appropriate host cells and study of the role of this factor in haematopoietic cell proliferation and differentiation.

Among the others, the most available host cells for production of recombinant human G-CSF is Escherichia coli, from which recombinant methionyl human granulocyte colony-stimulating factor (rmetHuG-CSF, Filgrastim) was isolated, purified, characterized and the use of rmetHuG-CSF in malignancies commonly associated with neutropenic infections during therapy, such as breast cancer, lymphoma, and leukemia was established ( L.M. Souza, T.C. Boone, J. Gabrilove, P.H. Lai, K.M. Zsebo, D.C. Murdock, V.R. Chazin, J. Bruszewski, H. Lu, K.K. Chen, J. Barendt, E. Platzer, M.A.S. Moore, R. Mertelsmann and K. Welte. Recombinant human granulocyte colony-stimulating factor: effects on normal and leukemic myeloid cells. Science, 232 (1986) 61-65); in "Filgrastim (r-metHuG-CSF) in Clinical Practice" ed. G.Morstyn, T. M. Dexter and M.A. Foote, 2nd ed, Marcel Dekker, Inc., New York, Basel, Hong Kong, 1998).

A number of processes for synthesis, isolation and purification of recombinant human G-CSF in E. coli have been developed. For large scale production and clinical use, there is a need for efficient protein purification procedures to remove host DNA and impurities such that a protein for human administration can meet the necessary requirements whilst retaining its biological activity. Accordingly, chromatographic processes and combinations thereof for the purification of G-CSF have been developed.

EP0347041 and EP0719860 describe a process for isolating and purifying G-CSF from a G-CSF producing microorganism comprising lysing the microorganism and separating insoluble material containing G-CSF from soluble proteinaceous material.

US5849883 describes a process for isolating and purifying G-CSF from a G-CSF producing microorganism. The process include steps of lysing the microorganism and separating G-CSF from soluble proteinaceous material; extracting the material with deoxycholate (optionally); solubilizing and oxidizing the G-CSF in the presence of a denaturant solubilizing agent and oxidizing agent; removing the denaturant solubilizing agent from the G-CSF; subjecting the G-CSF to ion-exchange chromatography; and recovering the purified G-CSF.

WO9853072 describes the production of human granulocyte colony-stimulating factor in a process comprising introducing a recombinant plasmid (pyHM-G-CSF) containing cDNA for hG-CSF into Escherichia coli which was developed to recombinant bacteria. The recombinant bacteria have high expression ability to 1.7g hG-CSF from 1L of culture media. The refolded and purified hG-CSF has comparable biological activity of G-CSF.

US5849883 describes the process for isolating and purifying granulocyte colony stimulating factor (G-CSF) from a G-CSF producing microorganism including the steps of lysing the microorganism and separating G-CSF from soluble proteinaceous material; extracting the material with deoxycholate (optionally); solubilizing and oxidizing the G-CSF in the presence of a denaturant solubilizing agent and oxidizing agent; removing the denaturant solubilizing agent from the G-CSF; subjecting the G-CSF to ion-exchange chromatography; and recovering the purified G-CSF. In accordance with this process, the denaturant solubilizing agent is Sarcosyl, the oxidizing agent is CuSO₄, denaturant solubilizing agent is removed using Dowex and ion exchange chromatography step is an anion-exchange chromatography step followed by cation-exchange chromatography

However, the processes available to date are generally time-, labour- and cost- consuming as well as being complicated. Moreover, they fail to provide stable results.

Accordingly, there is a need for improved purification protocols.

The structure of G-CSF mRNA (and corresponding cDNA) has been found to be unfit for the effective over-expression in *E. coli* (Devlin et al., 1988, Gene, 65: 13-22). The introduction of a few nucleotide modifications of 5'-end of cDNA sequence (including usage of alternative codons coding the same amino acids, but containing more A/T bases) has been shown to lead to effective translation of G-CSF mRNA and improved over-expression of G-CSF in E. *coli.* However, it is not possible to predict the effect of different codons on the expression level. Accordingly, there is a need for alternative mRNAs which have increased expression.

### SUMMARY OF THE INVENTION

The present invention provides a novel process for isolating and purifying rmetHuG-CSF from a G-CSF producing microorganism.

Accordingly, in a first aspect of the invention there is provided a process for isolating and purifying G-CSF from a G-CSF-producing microorganism comprising the steps:
a) lysing the microorganism and separating insoluble material comprising G-CSF from soluble proteinaceous material;
b) solubilising the G-CSF present in the insoluble material;
c) oxidizing the G-CSF in the presence of a pair oxidizing/reducing agent;
d) subjecting the solution to chromatography; and
e) recovering purified G-CSF

In one embodiment, the G-CSF is rmetHuG-CSF.

The G-CSF-producing microorganisms are grown in a suitable growth media, the composition thereof will depend upon the particular microorganism involved. Prior to lysis, the cells are harvested from the culture, and may be concentrated if necessary, by filtration, centrifugation, and other conventional methods. In a preferred embodiment of the present invention, the microorganism producing G-CSF is E. coli. Suitable strains of E. coli for production are known to the person skilled in the art.

In accordance with the procedures of the present invention, the cell membranes of the microorganisms are lysed using conventional techniques such as homogenization, sonication, or pressure cycling. Preferred methods include sonication or homogenization with a Rannie homogenizer.

After the cells have been lysed, the particulate matter containing rmetHuG-CSF is separated from the liquid phase of lysate and resuspended in appropriate buffer solution. The particulate matter may be optionally washed to remove any water soluble E. coli proteins therein.

Suitably, the rmetHuG-G-CSF in the particulate matter is solubilized in the presence of a solubilizing agent preferably under neutral pH conditions. The solubilizing agent is a chaotropic agent (i.e., a protein denaturant that dissociates hydrogen bonds and affects the tertiary structure of the proteins causing its unfolding) generally in an aqueous buffer solution.

Accordingly, in one embodiment, the G-CSF in the insoluble material is solubilized using a chaotropic agent.

Representative chaotropic agents include urea and guanidinium hydrochloride. Guanidinium hydrochloride is a stronger chaotropic agent and is preferred avoiding carbamoylation of polypeptide chain which may occur if concentrated urea solution is used.

Accordingly, in one embodiment, step b) includes incubation with guanidinium hydrochloride.

The concentration of the chaotropic agent will depend upon the particular agent that is used and the amount of cellular material present. In one embodiment, in step b) the concentration of guanidinium hydrochloride is from 3.0 to 3.2 M. In another embodiment, a guanidinium hydrochloride solution having a concentration of 6-7M is employed and most preferably a 7M guanidinium hydrochloride solution is employed.

The pH may be adjusted by adding suitable buffers, and preferably the pH will range from about 6.0 to about 8.0 and most preferably within pH range 6.8 - 7.2.

Following solubilization of the rmetHuG-CSF, insoluble particulate matter is separated and discarded.

Suitably, the soluble rmetHuG-CSF is oxidized in the presence of a pair reducing/oxidizing agent.

Suitable pair reducing/oxidizing agents include, for example, cysteine and cystine, dithiothreitol and its oxidized form *trans*-4,5-dihydroxy-1,2-dithiane, glutathione and oxidized glutathione (B. Fischer, I. Summer and P. Goodenough. Isolation, renaturation, and formation of disulfide bonds of eukaryotic proteins expressed in Escherichia coli as inclusion bodies. Biotechn. Bioengn.,41, 3-13 (1993); V. Lozanov, C. Guarnaccia, A. Patthy, S. Foot and S. Pongor. Synthesis and cystine/cysteine-catalyzed oxidative folding of the Amaranth α-amylase inhibitor. J.Peptide Res., 50, 65-72 (1997); Y-J. Li, D. M. Rothwarf, and H. A. Scheraga. An unusual adduct of dithiothreithol with a pair of cysteine residues of a protein as a stable folding intermediate. J. Amer. Chem. Soc., 120, 2668-2669 (1998)).

Advantageously, it has been found that the yield of correctly folded rmetHuG-CSF, that is, oxidized rmetHuG-CSF having the correct native conformation of disulfide bonds, is increased by facilitating rearrangement of disulfide bonds through the use of a glutathione redox buffer (glutathione and oxidized glutathione).

Accordingly, in one embodiment, the pair reducing/oxidizing agent used in step c) is a glutathione redox buffer (glutathione and oxidized glutathione).

The rmetHuG-CSF is oxidized by the oxidized glutathione and the presence of the reducing agent, glutathione, in the redox buffer substantially reduces the formation of incorrectly folded rmetHuG-CSF, that is rmetHuG-CSF with incorrect disulfide bonds.

The ratio of glutathione: oxidized glutathione in the redox buffer is readily ascertained by one of ordinary skill in the art. Preferably an excess of glutathione is employed, more preferably a ratio of from 5:1 to 20:1 on a molecular weight basis glutathione: oxidized glutathione is employed. Most preferably a 20:1 molar ratio of glutathione:oxidized glutathione is employed.

Suitably, the refolding of rmetHuG-CSF in the presence of redox buffer containing a pair reduced/oxidized glutathione is performed at an intermediate concentration of a chaotropic agent. In one embodiment, the refolding is at an intermediate guanidinium hydrochloride concentration. Suitably, the concentration of guanidinium hydrochloride is from 2.45M to 3.2M and most preferably in buffer solution containing from 3.0 to 3.2M of guanidinium hydrochloride.

Yield of rmetHuG-CSF refolding is strongly dependent on pH value of refolding buffer containing intermediate concentration of guanidinium hydrochloride and redox system.

Preferably the refolding reaction is performed by maintaining the pH of buffer solution from 6.5 to pH 8.0 and the most preferably from pH 7.15 to pH 7.30.

The resulting solution containing correctly folded rmetHuG-CSF is preferably centrifuged or filtrated to remove any remaining particulate matter and the resulting discarded solution is buffer exchanged to remove residual chaotropic agent such as guanidinium hydrochloride and constituents of redox system such as glutathione and oxidized glutathione.

Accordingly, in a further embodiment, the process of the invention additionally comprises separating the refolded G-CSF from chaotrope.

Suitably, where the chaotropic agent is guanidinium hydrochloride, it may be removed from a solution containing refolded rmetHuG-CSF by gel-filtration. Suitable gel-filtration media will be familiar to those skilled in the art. In one embodiment, the gel-filtration media is Sephadex G-25 chromatography media.

Suitably, the pH of the buffer solution is adjusted to a pH range suitable for gel-filtration. Where the gel-filtration media is Sephadex G-25, the pH is suitably adjusted to be between about 6.5 to 8.0 and preferably to 7.5.

The resulting mixture is filtered and the collected filtrate exposed for maturation. Suitably, the collected filtrate is exposed for about from 20 to 48 hours for correctly folded rmetHuG-CSF maturation. Correctly folded rmetHuG-CSF is then separated from any remaining contaminants employing chromatographic procedures.

It is preferred to employ ion exchange liquid chromatography to recover the purified rmetHuG-CSF. Suitable methods for ion exchange liquid chromatography will be familiar to those skilled in the art.

In one embodiment of the process of the invention, the chromatography in step d) is a two-step chromatography purification. Suitably, the chromatography is two-step ion exchange chromatography.

In a preferred mode of practice of this aspect of the invention, high yields of purified rmetHuG-CSF are recovered through the use of a DEAE-Sepharose ion exchange column which preferably operates at pH of a buffer of chromatography from 6.5 to 8.0, and most preferably at pH about 7.0 followed by separation using a SP-Sepharose which preferably operates at a pH of chromatography from about pH 5.2 to pH 5.6 and the most preferably at pH of 5.4.

Accordingly, in a preferred embodiment, the present invention provides a process for isolating and purifying rmetHuG-CSF from a G-CSF producing microorganism comprising:
1) lysing the microorganism and separating insoluble material containing rmetHuG-CSF from soluble proteinaceous material;
2) solubilizing the rmetHuG-CSF present in the insoluble material;
3) oxidizing the rmetHuG-CSF using oxidized glutathione in the presence of reduced glutathione;
4) separating of refolded rmetHuG-CSF from chaotrope
5) two-step chromatography purification of rmetHuG-CSF

In another embodiment, the invention provides a process comprising the steps of:
1) lysing the microorganism and separating insoluble material containing rmetHuG-CSF from soluble proteinaceous material;
2) solubilizing the rmetHuG-CSF present in the insoluble material;
3) oxidizing the rmetHuG-CSF in the presence of a pair of reducing and oxidizing agent
4) separating of rmetHuG-CSF from solubilizing agent,
5) time-dependent maturation of rmetHuG-CSF,
6) selectively separating correctly folded rmetHuG-CSF from incorrectly folded, aggregated and altered rmetHuG-CSF by anion-exchange chromatography followed by chromatography over strong cation-exchange column
7) transition of highly purified, correctly folded biologically active rmetHuG-CSF to its stable liquid formulation by chromatography over gel-filtration column
8) addition of stabilizing agent to defined formula of final liquid composition

In a further embodiment of the invention, the method comprises the further step of formulating the rmetHuG-CSF thus separated with pharmaceutically acceptable adjuvants. In one embodiment, formulation is through the addition of acetic acid buffer of pH 3.8-4.2 containing bulking and stabilizing additives to yield a final product that is acceptable for filling into appropriate delivery devices of definite dosage strengths for administration to patients.

The structure of G-CSF mRNA (and corresponding cDNA) has been found to be unfit for the effective over-expression in *E. coli* (Devlin et al., 1988, Gene, 65: 13-22). The introduction of a few nucleotide modifications of 5'-end of cDNA sequence (including usage of alternative codons coding the same amino acids, but containing more A/T bases) has been shown to lead to effective translation of G-CSF mRNA and improved over-expression of G-CSF in *E. coli.*

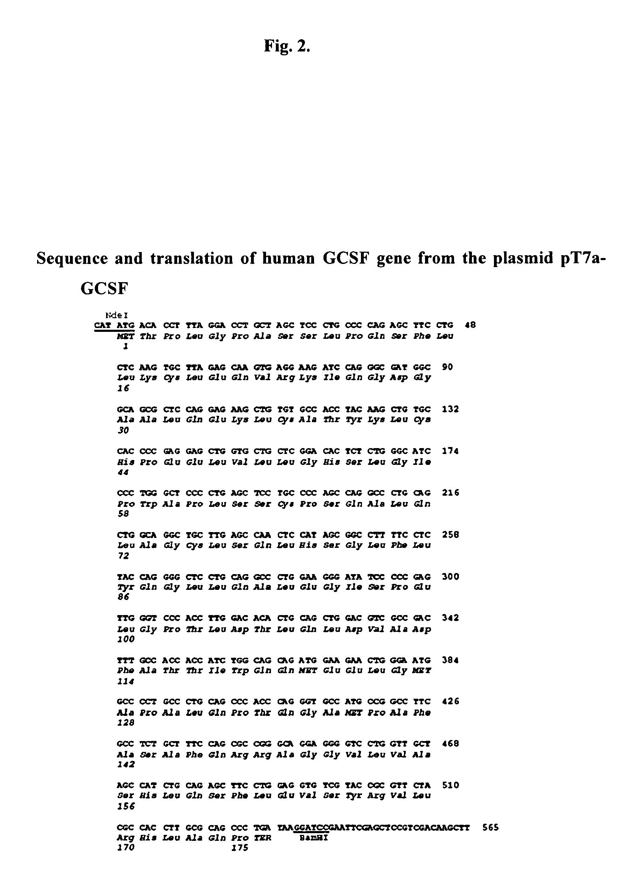

Accordingly, in one aspect, the present invention provides an isolated nucleic acid molecule having the nucleotide sequence set out in Figure 2.

In another aspect there is provided an expression plasmid comprising a nucleic acid molecule having the nucleotide sequence set out in Figure 2. Suitably, the expression has a strong T7 promoter. In one embodiment, the expression plasmid is pT7a-GCSF.

In another aspect there is provided a host cell comprising the expression plasmid in accordance with the invention. Suitably, the host cell is E.coli. In one embodiment, the E.coli is E.coli strain K802.

In a further aspect, there is provided a primer sequence for amplification of a modified hG-CSF sequence. Accordingly, in one aspect there is provided an isolated nucleic acid molecule having the sequence:
**CTGCATATGACACCTTTAGGACCTGCT**

### BRIEF DESCRIPTION OF THE DRAWINGS

**Figure 1** is the map of expression plasmid pT7a-GCSF
**Figure 2** is the sequence and translation of rmetHuG-CSF gene from the plasmid pT7a-GCSF
**Figure 3** is a schematic representation of the biosynthesis of rmetHuG-CSF by transformed E.coli producer.

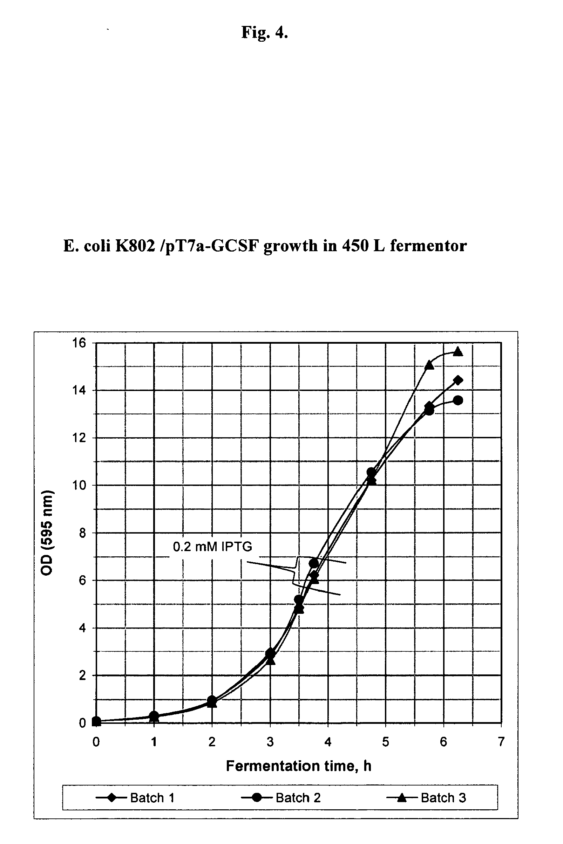

**Figure 4** represents E. coli K802/pT7a-GCSF growth in 450 L fermentor.

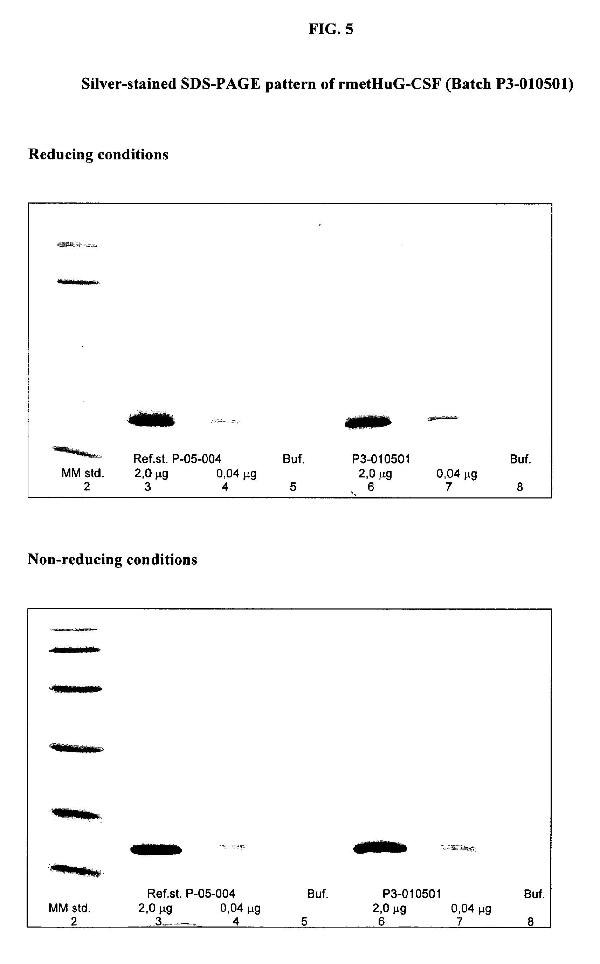

**Figure 5** represents the purity of processed according to this invention and formulated rmetHuG-CSF as judged by SDS-PAGE both under reducing and non-reducing conditions

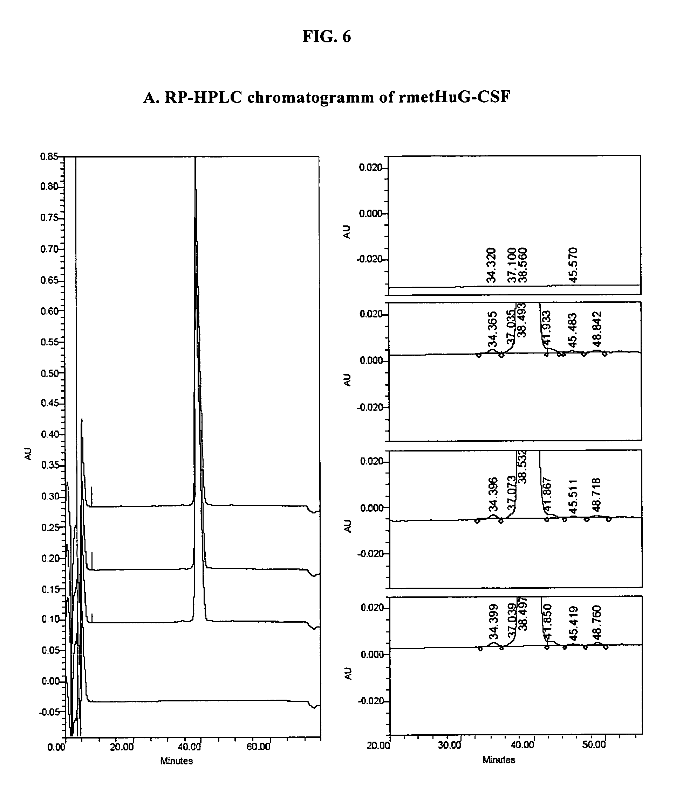

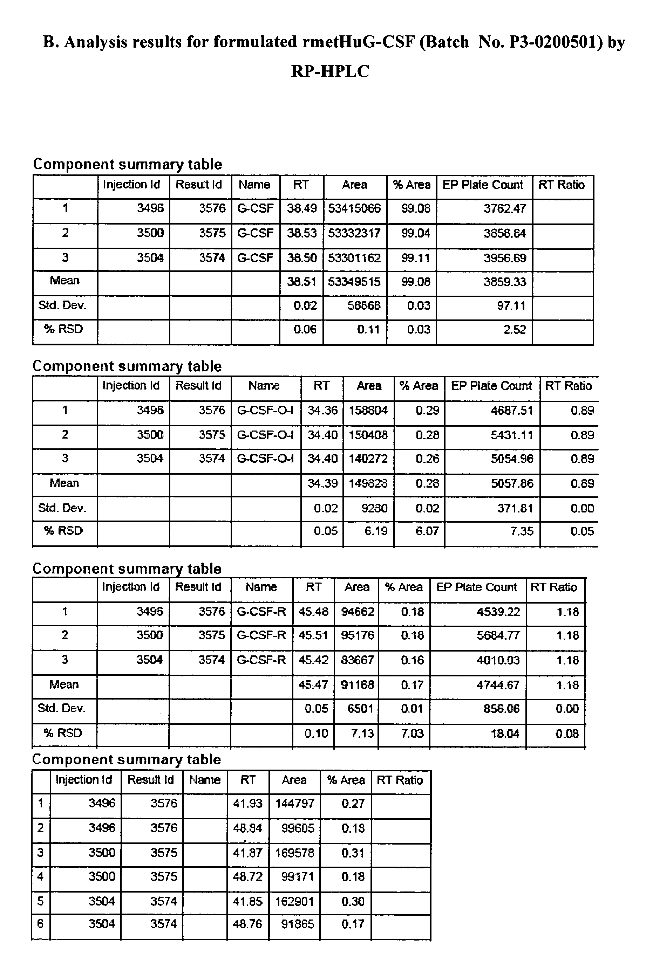

**Figure 6** represents the purity of processed according to this invention and formulated rmetHuG-CSF as judged by reversed-phase HPLC

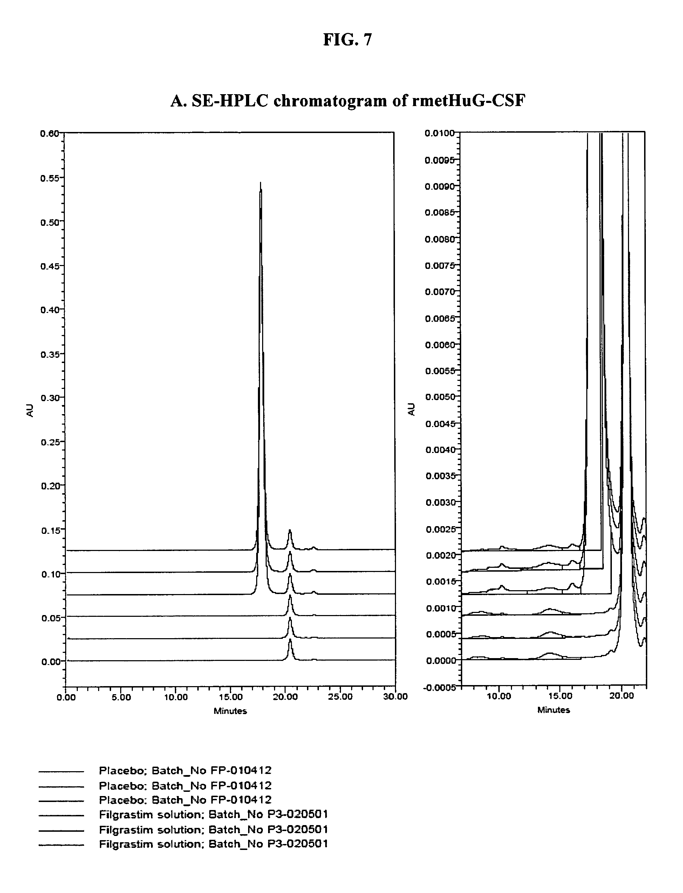

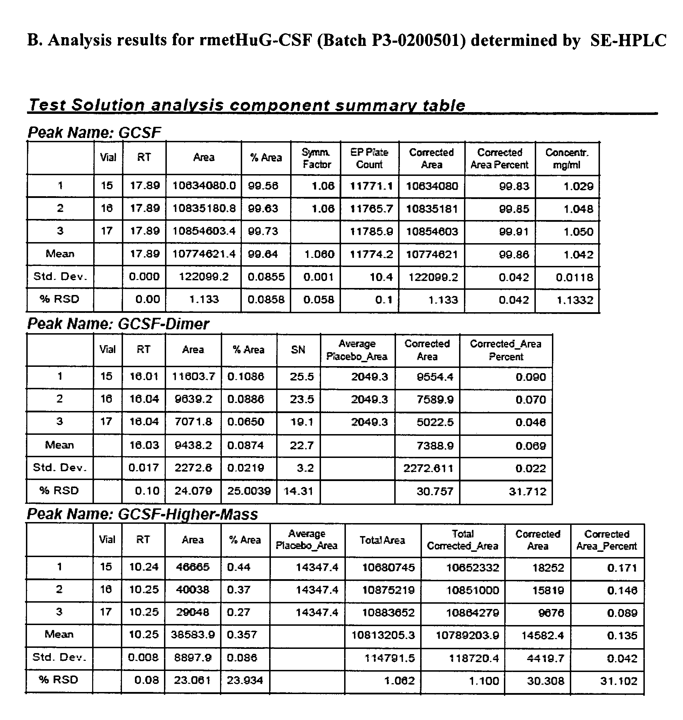

**Figure 7** represents the purity of processed according to this invention and formulated rmetHuG-CSF as judged by size-exclusion HPLC

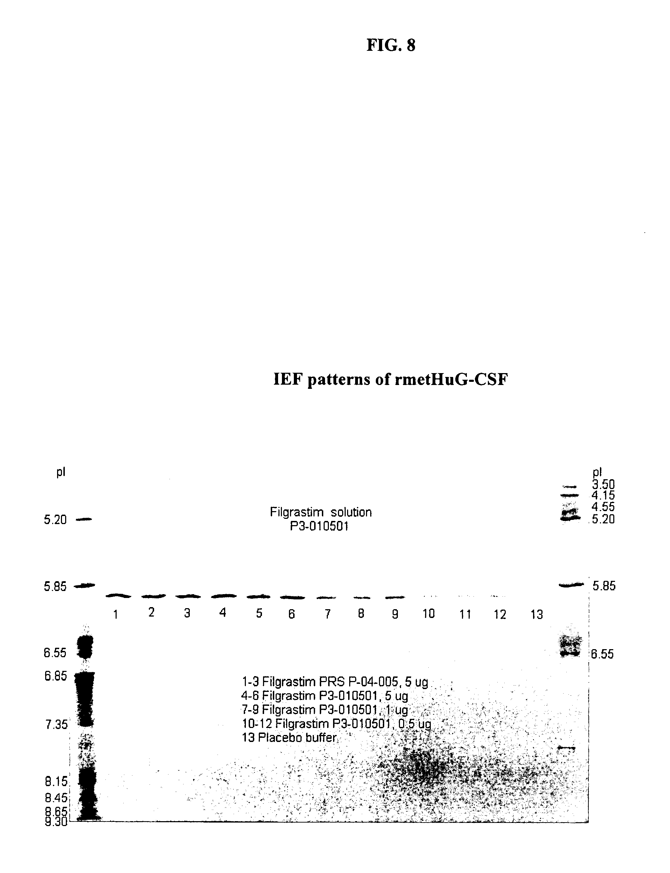

**Figure 8** represents the purity of processed according to this invention and formulated rmetHuG-CSF as judged by isoelectric focusing method

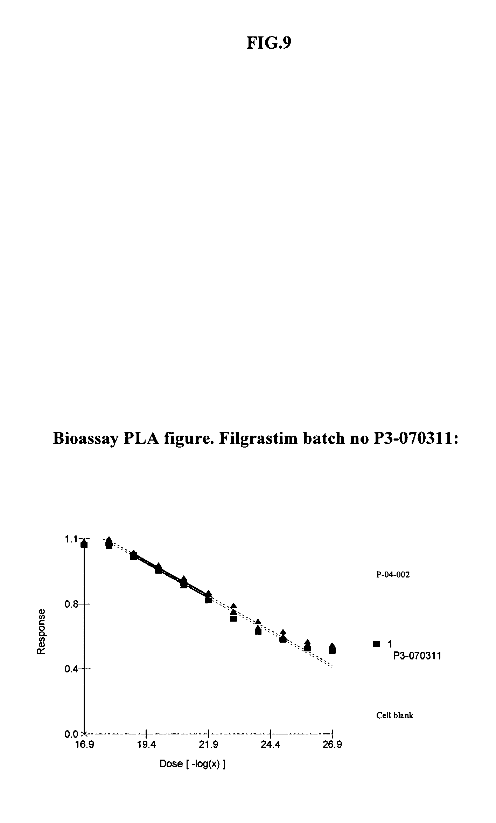

**Figure 9** represents the biological activity assay of purified according to this invention and formulated rmetHuG-CSF

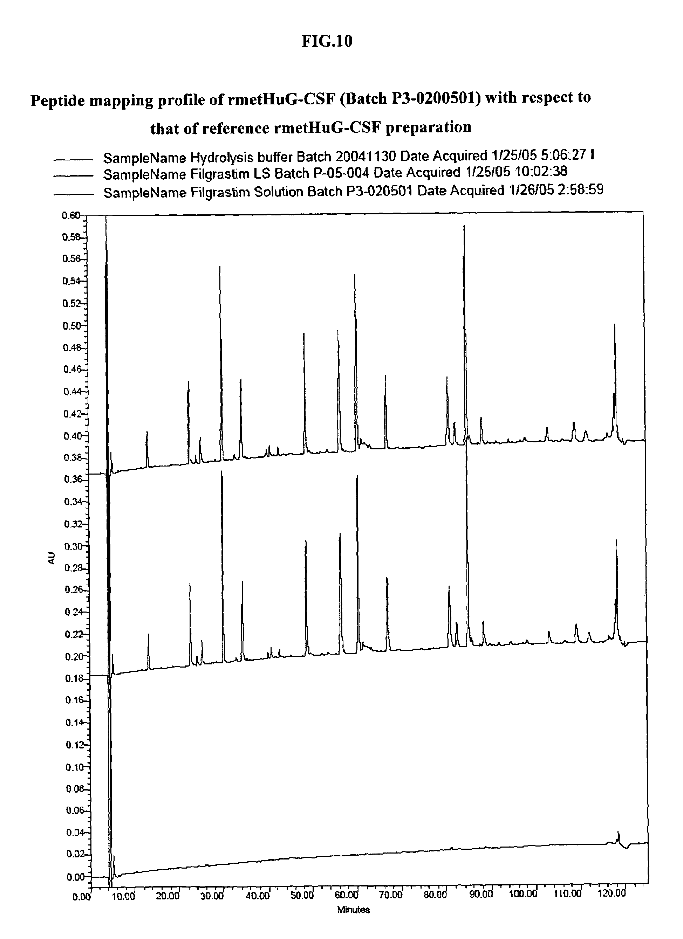

**Figure 10** represents the identity testing of purified according to this invention and formulated rmetHuG-CSF by peptide mapping

### DETAILED DESCRIPTION OF THE INVENTION

The practice of the present invention will employ, unless otherwise indicated, conventional techniques of chemistry, molecular biology, cell biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, IRL Press; and, D. M. J. Lilley and J. E. Dahlberg, 1992, Methods of Enzymology: DNA Structure Part A: Synthesis and Physical Analysis of DNA Methods in Enzymology, Academic Press. Each of these general texts is herein incorporated by reference.

As used herein the term "rmetHuG-CSF" refers to a protein that is produced by a microorganism that has been transformed with a hG-CSF gene or modification thereof that encodes a protein having (1) an amino acid sequence that is at least substantially identical to the amino acid sequence of native human rmetHuG-CSF and (2) biological activity that is common to native human G-CSF.

"Substantial identical amino acid sequence" means that the sequences are identical or differ by one or more conservative amino acid mutation or alteration (i.e., deletions, additions, substitutions) that do not produce an adverse functional dissimilarity between the synthetic protein and native human G-CSF.

By "conservative mutation" is meant mutations to amino acid residues that are conservative in terms of the amino acid characteristics compared to the amino acid residue indicated. Amino acid characteristics include the size of the residue, hydrophobicity, polarity, charge, pK-value, and other amino acid characteristics known in the art.

As used herein the term "rmetHuG-CSF producing microorganism" refers to a microorganism that has been genetically engineered to produce a protein that possesses biological activity associated with human G-CSF. As used herein the term "biological activity of rmetHuG-CSF" includes therapeutic activity of human G-CSF.

A rmetHuG-CSF producing microorganism can be generated by introducing a nucleic acid molecule encoding G-CSF. Suitably the G-CSF-encoding sequence may be part of an expression vector. Preferably, the G-CSF sequence is the codon modified G-CSF sequence described herein.

### EXPRESSION VECTOR

The terms "plasmid", "vector system" or "expression vector" means a construct capable of *in vivo* or *in vitro* expression. In the context of the present invention, these constructs may be used to introduce genes encoding enzymes into host cells.

Preferably, the expression vector is incorporated into the genome of a suitable host organism. The term "incorporated" preferably covers stable incorporation into the genome.

The nucleotide sequences described herein including the nucleotide sequence of the present invention may be present in a vector in which the nucleotide sequence is operably linked to regulatory sequences capable of providing for the expression of the nucleotide sequence by a suitable host organism.

The vectors for use in the present invention may be transformed into a suitable host cell as described below to provide for expression of a polypeptide of the present invention.

The choice of vector e.g. a plasmid, cosmid, or phage vector will often depend on the host cell into which it is to be introduced. Suitable vectors include pUC57/T and pT7a as described herein.

The vectors for use in the present invention may contain one or more selectable marker genes- such as a gene, which confers antibiotic resistance e.g. ampicillin, kanamycin, chloramphenicol or tetracyclin resistance. Alternatively, the selection may be accomplished by co-transformation (as described in WO91/17243).

Vectors may be used *in vitro,* for example for the production of RNA or used to transfect, transform, transduce or infect a host cell.

Thus, in a further embodiment, the invention provides a method of making nucleotide sequences of the present invention by introducing a nucleotide sequence of the present invention into a replicable vector, introducing the vector into a compatible host cell, and growing the host cell under conditions which bring about replication of the vector.

The vector may further comprise a nucleotide sequence enabling the vector to replicate in the host cell in question. Examples of such sequences are the origins of replication of plasmids pUC19, pACYC177, pUB110, pE194, pAMB1, pIJ702 and pET11.

### REGULATORY SEQUENCES

In some applications, the nucleotide sequence for use in the present invention is operably linked to a regulatory sequence which is capable of providing for the expression of the nucleotide sequence, such as by the chosen host cell. By way of example, the present invention covers a vector comprising the nucleotide sequence of the present invention operably linked to such a regulatory sequence, i.e. the vector is an expression vector.

The term "operably linked" refers to a juxtaposition wherein the components described are in a relationship permitting them to function in their intended manner. A regulatory sequence "operably linked" to a coding sequence is ligated in such a way that expression of the coding sequence is achieved under condition compatible with the control sequences.

The term "regulatory sequences" includes promoters and enhancers and other expression regulation signals.

The term "promoter" is used in the normal sense of the art, e.g. an RNA polymerase binding site.

Enhanced expression of the nucleotide sequence encoding G-CSF invention may also be achieved by the selection of heterologous regulatory regions, e.g. promoter, secretion leader and terminator regions.

Preferably, the nucleotide sequence according to the present invention is operably linked to at least a promoter.

Examples of suitable promoters for directing the transcription of the nucleotide sequence in suitable bacterial host cells are well known in the art.

### HOST CELLS

The term "host cell" - in relation to the present invention includes any cell that comprises either the nucleotide sequence or an expression vector as described above and which is used in the recombinant production of an enzyme having the specific properties as defined herein or in the methods of the present invention.

Thus, a further embodiment of the present invention provides host cells transformed or transfected with a nucleotide sequence that expresses G-CSF as described herein. The cells will be chosen to be compatible with the said vector and may for example be prokaryotic (for example bacterial), fungal, yeast or plant cells. Preferably, the host cells are prokaryotic cells.

Examples of suitable bacterial host organisms are gram positive or gram negative bacterial species.

The genotype of the host cell may be modified to improve expression.

Examples of host cell modifications include protease deficiency, supplementation of rare tRNA's, and modification of the reductive potential in the cytoplasm to enhance disulphide bond formation.

For example, the host cell *E. coli* may over-express rare tRNA's to improve expression of heterologous proteins as exemplified/described in Kane (Curr Opin Biotechnol (1995), 6, 494-500 "Effects of rare codon clusters on high-level expression of heterologous proteins in E.coli"). The host cell may be deficient in a number of reducing enzymes thus favouring formation of stable disulphide bonds as exemplified/described in Bessette (Proc Natl Acad Sci USA (1999), 96, 13703-13708 " Efficient folding of proteins with multiple disulphide bonds in the Escherichia coli cytoplasm").

Suitable bacterial host cells include E. coli. Suitably E. coli strains include HB101, JM109 and K802, as described herein.

### PURIFIED

By "isolated" or "purified" is meant that the G-CSF protein is in a relatively pure state - e.g. at least about 90% pure, or at least about 95% pure or at least about 98% pure.

For use in the process of the present invention, various separation media can be used.

### Various gel filtration supports can be used and are selected from the group comprising:

Sephacryl S-200HR, Sephacryl S-100HR, Superose 12, Superose 6, Superdex 75, TSKgel G-2500PW, TSK gel G-3000 PW, Bio-Gel P-60, Bio-Gel P-100 etc. Preferably, Superdex 75 is used.

Various cationic exchange chromatography supports can be used and may be selected from the group comprising: SP Sepharose FF, SP Sepharose HP, CM Sepharose FF, TSKgel SP-5PW, TSK gel SP-5PW-HR, Toyopearl SP-650M, Toyopearl SP-650S,Toyopearl SP-650C, Toyopearl CM-650M,Toyopearl CM-650S, Macro-Prep High S support, Macro-Prep S support, Macro-Prep CM support etc

### THERAPEUTIC AND FORMULATION

The term 'therapeutical effective amount' used herein refers to the amount of biologically active G-CSF which has the therapeutic effect of biologically active G-CSF.

Suitable pharmaceutically acceptable auxiliary substances for use in formulating an isolated or purified G-CSF using processes in accordance with the invention include suitable diluents, adjuvants and/or carriers useful in G-CSF therapy.

Biologically active G-CSF obtained by using the process of the present invention, particularly when performing the additional steps of cationic exchange chromatography and gel filtration, can be used for preparation of medicaments.

Such medicaments are indicated for a wide range of indications which will be familiar to the skilled person and include: neutropenia and neutropenia-related clinical sequelae, reduction of hospitalisation for febrile neutropenia after chemotherapy, mobilisation of hematopoietic progenitor cells, as alternative to donor leukocyte infusion, chronic neutropenia, neutropenic and non-neutropenic infections, transplant recipients, chronic inflammatory conditions, sepsis and septic shock, reduction of risk, morbidity, mortality, number of days of hospitalisation in neutropenic and non-neutropenic infections, prevention of infection and infection-related complications in neutropenic and non-neutropenic patients, prevention of nosocomial infection and to reduce the mortality rate and the frequency rate of nosocomial infections, enteral administration in neonates, enhancing the immune system in neonates, improving the clinical outcome in intensive care unit patients and critically ill patients, wound/skin ulcers/burns healing and treatment, intensification of chemotherapy and/or radiotherapy, pancytopenia, increase of anti-inflammatory citokines, shortening of intervals of high-dose chemotherapy by the prophylactic employment of filgrastim, potentiation of the antitumour effects of photodynamic therapy, prevention and treatment of illness caused by different cerebral disfunctions, treatment of thrombotic illness and their complications and post irradiation recovery of erythropoiesis. It can be also used for treatment of all other illnesses, which are indicative for G-CSF.

A pharmaceutical composition containing the pure and biologically active G-CSF obtained by the process of the invention can thus be administered, in a manner known to those skilled in the art, to patients in a therapeutical amount which is effective to treat the above mentioned diseases.

The present invention will now be described with reference to the following examples.

### EXAMPLES:

### EXAMPLE 1. DEVELOPMENT OF BACTERIAL EXPRESSION STRAIN PRODUCING HUMAN RECOMBINANT G-CSF (RMETHUG-CSF)

Cloning and expression of human G-CSF gene as well as construction of the bacterial strain producing recombinant G-CSF protein was achieved using following steps: cloning of cDNA gene, modification of the DNA sequence of the gene to optimize the expression possibility in *Escherichia coli,* construction of expression plasmid, transformation of selected plasmid into suitable *E. coli* strain and selection of expression / induction conditions. The structure of G-CSF mRNA (and corresponding cDNA) was found not fitting to the effective over-expression in *E. coli* (Devlin et al., 1988, Gene, 65: 13-22). Few nucleotide modifications of 5'-end of cDNA sequence (including usage of alternative codons coding the same amino acids, but containing more A/T bases) could lead to effective translation of G-CSF mRNA and as a consequence of these changes over-expression of G-CSF in *E. coli* could be achieved.

The *E. coli* strain producing recombinant human G-CSF was constructed as described below.

### Methods

Methods and protocols used in cloning and expression of human GCSF are from: Sambrook J., Frich E.F., Maniatis T., Molecular cloning: A Laboratory Manual, the second ed., CSH Laboratory, Cold Spring Harbor, 1989. Current Protocols in Molecular Biology, vol. 1-3 (Ausubel F.M. et al., ed.) John Wiley & Sons, Inc., Brooklyn, New York, 1994-1998.

All operations with enzymes, DNA and protein markers are fulfilled according to the manufacturer's instructions (mainly Fermentas).

### Genotype of E. coli strains used

**HB101 -** F⁻ thi1 hsd20 (r_{b}⁻ m_{b}⁻) supE44 recA13 ara14 leuB6 proA21acY1 rpsL20(Str^{r}) xyl5 mtl5 galK2
**JM109 - F'** traD36 proAB lacI^{q} Δ(lacZ)M15 / end A1 gyrA96(Nal^{c}) recA1 thi hsdR17 (rₖ⁻ mₖ⁻) relA1 supE44 Δ(lac-proAB)
**K802 -** F⁻ e14⁻(McrA⁻) lacY1 or Alac(I-Y)6 supE44 galK2 ga1T22 rfbD1 metB1 mcrB1 hsd S3 (rₖ⁻ mₖ⁺)

The cDNA gene of G-CSF was obtained as follows:
mRNA prepared from human leukocytes was used to synthesize cDNA from oligo(dT) primer. G-CSF specific cDNA was amplified by PCR using primers complementary to 5'- and 3'-ends of mature G-CSF cDNA sequence:
   5' ACC CCC CTG GGC CCT GGC (GCSF 5'-end, sense)
   5' TCA GGG CTG CGC AAG GTG (GCSF 3'-end, anti-sense)

The PCR fragment obtained was ligated into the pUC57/T (Fermentas) plasmid and sequenced (the sequence was found identical with published by Souza L. et al., 1986, Science 232: 61-66*,* and Nagata S. et al., 1986, Nature, 319: 415-418). Plasmid pUC57-GCSF was used as initial for further cloning and expression experiments.

After unsuccessful attempts to express in *E. coli* cDNA nucleotide sequence of the unmodified cDNA gene of human G-CSF, it was assumed that the high G/C nucleotide content in the region encoding the N-terminal portion of the G-CSF protein may cause an insufficient expression of the G-CSF gene (Devlin et al., 1988, Gene, 65: 13-22). Thus, an approach to increase the A/T content within the 5'-end of the coding region of the gene without altering the predicted amino acid sequence was applied. The modifications of the nucleotide structure that have been made are shown in Table 1.

**Table 1. DNA sequence changes in the cDNA gene of human G-CSF**

| **Plasmid** | **G-CSF gene sequence** |
|---|---|
| pUC57-GCSF (cDNA gene) | Thr Pro Leu Gly Pro Ala.. ACC CCC CTG GGC CCT GCC... |
| pT7a-GCSF (modified nucleotide sequence) | Met Thr Pro Leu Gly Pro Ala... *ATG ACA CCT TTA GGA CCT GCT...* |

Modification of the cDNA gene, together with the introduction of cleavage sites for restriction endonucleases *NdeI* and *BamHI* at the ends of the fragment, was achieved by PCR amplification using primers C and D, complementary to 5'- and 3'- terminus of the human G-CSF, respectively (Table 2). Primer C had six nucleotides (underlined) changed in comparison to the natural sequence of GCSF, to introduce the required modifications into the gene. 25 cycles of PCR (94°C, 1 min.; 58°C, 1 min.; 72°C, 1 min.) were carried out using *Taq* polymerase. This enzyme during PCR produces amplified DNA fragment with single protruding dA nucleotide in the 3'-ends of the fragment.

**Table 2. Primers used for PCR amplification of G-CSF gene insert**

| **Primer** | **Sequence 5'→ 3'** |
|---|---|
| **C** (modified 5'-terminus of G-CSF) | CTGCATATGACACCTTTAGGACCTGCT |
| **D** (3'-terminus of G-CSF) | CTGGGATCCTTATCAGGGCTG |

The amplified fragment was ligated into pUC57/T vector (Fermentas), which was designed for direct ligation of amplified DNA fragments with protruding 3'-dA-ends. E. *coli* JM109 cells were transformed with the ligation mixture, and screened for the recombinant plasmids, harbouring the desired structure insert, using the PCR procedure with primers C and D, complementary to 5'- and 3'- coding region of G-CSF (Table 2). Several clones yielding specific 540bp DNA fragment were selected, plasmid DNA was isolated and digested with restriction endonucleases *NdeI*+*BamHI.* Inserts from few positive clones have been sequenced and clones with expected DNA sequence (pUC57-GCSF plasmids) were picked for G-CSF expression vector construction.

To express the human G-CSF sequence in E. *coli,* the modified coding region have been shuttled into the expression vector pT7a, containing synthetic T7 early promoter (the sequence of promoter is similar to the sequences mentioned in publications M. Lancer and H. Bujard, PNAS, 1988, vol. 85, pp. 8973-8977 and Patent application EP0303925.)

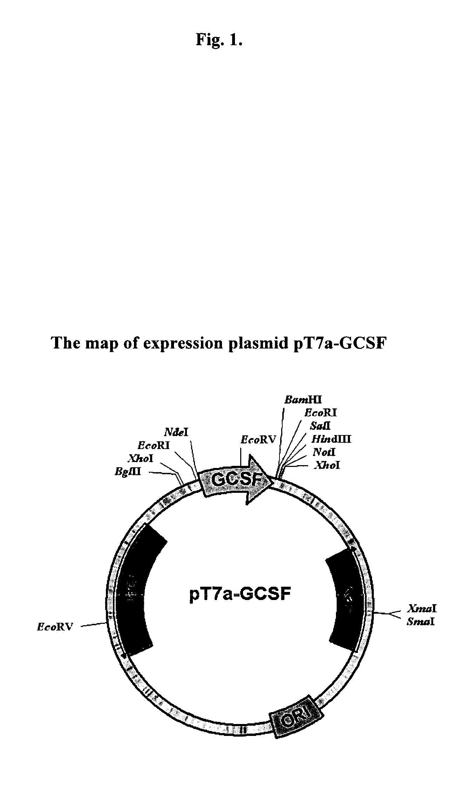

For this purpose, the pUC57-GCSF plasmid from the selected positive clone have been digested with restriction endonucleases *NdeI*+*BamHI,* and the resulting fragment carrying GCSF gene was purified from agarose gel. In analogous manner, vector pT7a was treated to yield the linear form of DNA suitable for cloning into *NdeI*+*BamHI* sites. *NdeI*+*BamHI* fragments of both G-CSF gene and pT7a vector were ligated and transformed into *E. coli* HB 101 cells. Screening for the proper recombinant plasmid pT7a-GCSF was fulfilled using the PCR and restriction analysis. Complete sequencing of the structural portion of the G-CSF gene was performed on the plasmid pT7a-GCSF (Fig.1). Nucleotide sequence of the gene and corresponding amino acid sequence of rmetHuG-CSF is presented in Fig.2.

The plasmid pT7a-GCSF was transformed into expression host strain *E. coli* K802. A few of selected colonies were inoculated into LB media, cultivated briefly and induced with IPTG to screen electrophoretically for the presence of polypeptide with molecular weight ~18.6 kDa specific to rmetHuG-CSF protein. SDS-PAAG electrophoresis analysis of induced cell lysates proved over-expression of GCSF. In addition, using fractionation (centrifugation) of the cell lysates prepared after induction, it was shown that rmetHuG-CSF protein is accumulated in the form of inclusion bodies found in the insoluble fraction of cell lysates obtained by sonication of total cellular suspension of the strain *E. coli* K802 pT7a-GCSF.

### EXAMPLE 2. DESCRIPTION OF THE BIOSYNTHESIS PROCESS

The *E. coli K802* /*pT7a-GCSF* strain was cultivated in media of the following composition (g/L): casamino acids (BD) - 12,0; yeast extract (BD) - 3,0; ammonium chloride (Merck) - 1,0; magnesium sulfate heptahydrate (Merck) - 0,5; di-Sodium hydrogen phosphate (Merck) - 4,706; potassium dihydrogen phosphate (Merck) - 4,54; D(+)-Glucose monohydrate (Merck) - 10,0.

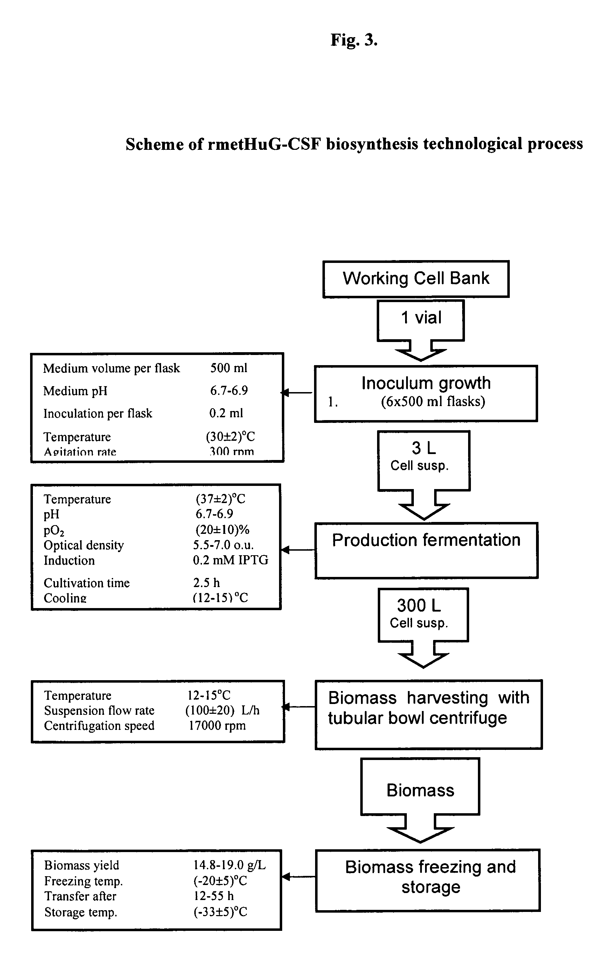

Full scheme of the rmetHuG-CSF biosynthesis technological process is presented in Fig. 3.

Six Erlenmeyer flasks, containing 500 ml of sterile medium were inoculated with 0.2 ml of stock culture E. coli K802/pT7a-GCSF from qualified WCB. The flasks were incubated on rotating shaker at agitation speed 300 rpm and 30°C temperature for 14-15 hours.

Sample for evaluation of medium sterility was taken before inoculation, culture purity - after cultivation.
Fermentor (450 L total/300 working volume) was inoculated with 3 L of culture obtained in the flasks. Fermentation was performed at automatically controlled temperature (37±2)°C, pH (6.7-6.9) and pO₂ saturation (20±10) % parameters. Induction of rmetHuG-CSF biosynthesis was performed at optical density of 5.5-7.0 optical units (λ-595 nm) with isopropyl-β-D-thiogalactopyranoside (IPTG, Roth) to make final concentration 0.2 mM and fermentation continued for another 2.5 hours at the same conditions (Fig. 2). After cell suspension in the fermentor cooling down to (12-15)°C temperature transferring with peristaltic pump (flow rate 100±20 L/h) into the tubular bowl centrifuge for biomass harvesting was started.

Sample for evaluation of medium sterility was taken before inoculation, culture purity - after cultivation, plasmid stability - before induction.

Cell suspension was centrifuged at speed of 17 000 rpm. Cell suspension temperature (12-15)°C was kept all over centrifugation.
Samples for supernatant turbidity (λ-595 nm) were taken at every half an hour.

Harvested biomass was collected into polyethylene bags and frozen at (-20±5)°C temperature. After 12-55 hours the biomass was transferred into (-33±5)°C refrigerator for storage.

### EXAMPLE 3. DESCRIPTION OF THE DOWN-STREAM PROCESS

Cell paste containing rmetHuG-CSF in transformed E. coli cells, such as obtained from Example 2, was dispersed in tank with agitator in 53 parts 0.1 M Tris buffer (pH 6.65) at a temperature of approximately 5 DEG C. The suspension was passed through a Rannie high pressure homogenizer two times. The homogenate was maintained at a temperature of approximately 5 DEG C. The homogenate was diluted to 55 parts 0.025 M Tris buffer (pH 7.05), mixed with IKA mixer and the resulting mixture was centrifuged at a temperature of 5 DEG C. The supernatant was decanted and the remaining residue was resuspended with IKA mixer in 0.025 M Tris buffer (pH 7.05) to yield a mixture having a final volume of 55 parts water. The resulting mixture was centrifuged at a temperature of 5 DEG C and the supernatant was decanted and the remaining residue was suspended with IKA mixer in water to yield a mixture having a final volume of 55 parts water. The resulting mixture was centrifuged at a temperature of 5 DEG C and the supernatant was decanted. In the resulting residue was suspended with IKA homogenizer in 1 parts of 0.58M Tris (pH 7.1) and 28 parts of 7.25M guanidinum hydrochloride for 3 hours at a temperature of 5 DEG C. The resulting mixture was filtered by passing through 0.45 µm tangential flow filtration membrane and filtrate was collected. To the filtrate was added a solution containing 0.00032 parts of oxidized glutathione and 0.0031 parts of reduced glutathione in 40 parts of 20 mM Tris (pH 7.15). The resulting mixture was adjusted to pH 7.22 and maintained at approximately 5 DEG C for 15.5 hours. The solution was loaded onto a Sephadex G-25 at 5 DEG C and eluted with 10 mM Tris (pH 7.5). The eluate from Sephadex G-25 after 3.5 hours exposing was filtered by passing through 0.22 µm tangential flow filtration membrane and filtrate was collected. The filtrate was exposed for 20-48 hours and then loaded onto a DEAE-Sepharose column at 5 DEG C and eluted with 20 mM Tris, 50 mM NaCl (pH 7.5). The conductivity of the eluent was adjusted to 3.2 mS/cm with 20 mM Sodium acetate (pH 5.0). The pH of the eluent was adjusted to pH 5.4 with 10% NaOH. The standardized eluate from the DEAE-Sepharose column containing rmetHuG-CSF was chromatographed on to a SP-Sepharose column at 5 DEG C. Biologically active rmetHuG-CSF was eluted from the column with a linear gradient from 20 mM Sodium acetate, 20 mM NaCl (pH 5.4) to 20 mM Sodium acetate, 250 mM NaCl (pH 5.4). The eluate collected was chromatographed on to a Sephadex G-25 column at 5 DEG C. The rmetHuG-CSF was eluted off the column with 10 mM Sodium acetate (pH 4.0). The eluate containing highly purified rmetHuG-CSF was diluted with buffer to a final concentration of 1.05 mg/ml with 10 mM Sodium acetate, 1% polysorbate and 0.05 parts of sorbitol (pH 4.0).

All publications mentioned in the above specification, and references cited in said publications, are herein incorporated by reference. Various modifications and variations of the described methods and system of the present invention will be apparent to those skilled in the art without departing from the scope and spirit of the present invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. A process for isolating and purifying G-CSF from a G-CSF-producing microorganism comprising the steps:
a) lysing the microorganism and separating insoluble material comprising G-CSF from soluble proteinaceous material;
b) solubilising the G-CSF present in the insoluble material;
c) oxidizing the G-CSF in the presence of a pair oxidizing/reducing agent;
d) subjecting the solution to chromatography; and
e) recovering purified G-CSF

2. A process as claimed in claim 1 wherein the G-CSF is rmetHuG-CSF.

3. A process as claimed in claim 1 or claim 2 wherein the pair oxidizing/reducing agent is a pair of oxidized/reduced glutathiones.

4. A process according to claim 3 wherein the molar ratio of oxidized and reduced glutathione is 1:20

5. A process as claimed in any of claims 1 to 4 wherein the G-CSF in the insoluble material is solubilized using a chaotropic agent.

6. A process as claimed in claim 5 wherein step c) is at an intermediate concentration of a chaotropic agent.

7. A process as claimed in claim 5 or claim 6 additionally comprising separating the refolded G-CSF from chaotrope.

8. A process as claimed in claim 7 wherein the refolded G-CSF is separated from chaotrope by gel-filtration.

9. A process as claimed in claim 8 wherein the gel-filtration column is Sephadex G-25.

10. A process as claimed in any of claims 1 to 9 wherein the chromatography in step d) is a two-step chromatography purification.

11. A process as claimed in claim 10 wherein the chromatography is two-step ion exchange chromatography.

12. A process as claimed in any of claims 1 to 11 wherein the solubilising step b) is using guanidinium hydrochloride.

13. A process according to claim 12 wherein in step b) the concentration of guanidinium hydrochloride is from 3.0 to 3.2 M.

14. A process as claimed in any of claims 1 to 13 wherein in step c) the pH is 7.15 - 7.30.

15. A process as claimed in any of claims 1 to 14 wherein step d) is DEAE-Sepharose followed by SP-Sepharose column.

16. A process as claimed in claim 15 wherein SP-Sepharose column separation is conducted at a pH of from 5.2 to pH 5.6.

17. A process according to any of the preceding claims wherein the microorganism producing G-CSF is E. coli.

18. A process for isolating and purifying rmetHuG-CSF from a G-CSF producing microorganism comprising:
a) lysing the microorganism and separating insoluble material containing rmetHuG-CSF from soluble proteinaceous material;
b) solubilizing the rmetHuG-CSF present in the insoluble material;
c) oxidizing the rmetHuG-CSF using oxidized glutathione in the presence of reduced glutathione;
d) separating of refolded rmetHuG-CSF from chaotrope
e) two-step chromatography purification of rmetHuG-CSF

19. A process according to any of claims 1 to 18 further comprising formulation of purified G-CSF.

20. An isolated nucleic acid molecule having the nucleotide sequence set out in Figure 2.

21. An expression plasmid comprising a nucleic acid molecule as claimed in claim 20.

22. An expression plasmid as claimed in claim 21 wherein the expression plasmid is pT7a-GCSF.

23. A host cell comprising the expression plasmid as claimed in claim 21 or claim 22.

24. A host cell as claimed in claim 23 wherein the host cell is E.coli.

25. A host cell as claimed in claim 23 wherein the host cell is E.coli strain K802.

26. An isolated nucleic acid molecule having the sequence:
CTGCATATGACACCTTTAGGACCTGCT
